# EUROPEAN PATENT APPLICATION

(11) **EP 0 672 420 A1**
(43) Date of publication of application: **20.09.1995**
(21) Application number: 94908813.2
(22) Date of filing: 10.08.1993
(51) Int. Cl.: A61K 37/30

(54) **WOUND REMEDY**

(30) Priority: 26.08.1992 JP 227325/92; 13.10.1992 JP 274126/92
(71) Applicant: Asahi Kasei Kogyo Kabushiki Kaisha, Osaka-shi Osaka 530 (JP)
(72) Inventor: ASANO, Toshio, Mishima-shi, Shizuoka 411 (JP); SAIKI, Kenji, Tagata-gun, Shizuoka 410-23 (JP)
(74) Representative: BROOKES & MARTIN
(86) International application number: JP9301123
(87) International publication number: WO9405320

(57) **Abstract**

A wound remedy containing as the active ingredient a peptide derivative related to a calcitonin gene, represented by general formula (I), or a salt thereof, wherein R represents Ala-NH-, Ser-NH-, NH₂ or H; Y represents S or CH₂; A represents Asp or Asn; B represents Gly, Asp or Glu; C represents Leu or Phe; D represents Val or Met; E represents Val or Gly; F represents Asn, Ser or Asp; G represents Asn or Asp; and I represents Lys or Glu. The administration of the remedy to the affected part intravenously, intramuscularly or subcutaneously or as an external preparation serves to promote the reepithelialization, granulation and arterialization of the wound, and remarkably inhibit inflammatory cellular infiltration, thus being useful for curing the wound.

R-CH —— CH₂ —— Y —— Y —— CH₂
  |                                    |
  CO- A -Thr-Ala-Thr-NHCH-CO-Val-
Thr-His-Arg-Leu-Ala- B - C -Leu-
Ser-Arg-Ser-Gly-Gly- D - E -Lys-      (I)
 F -Asn-Phe-Val-Pro-Thr- G -Val-
Gly-Ser- I -Ala-Phe-NH₂

## Description

### Detailed explanation of the invention

### Field of the invention

The present invention relates to a wound healing remedy for treatment of human injury caused by any reasons, for example operation, traffic accident and long term confining in bed. More particularly the present invention relates to a wound remedy containing a calcitonin gene related peptide represented by the formula hereinbelow as an active ingredient.

### Prior arts

Treatment of human injury and pressure sore caused by the reasons, for example operation, traffic accident and long term confining in bed, has been made conventionally by at first disinfecting the injured parts, suturing and waiting spontaneous healing of the injured parts. However such the conventional treatment progress requires long term for repair together with suffering pain in patients and accompanying deterioration by bacterial infection. Artificial stimulation of the healing is required in the injury, however nosotropic treatments such as administering analgesics for complaining pain and antibiotics for possible infections are the only measures in the present days treatments. Therefore wound healing remedy repairing injured parts has been desired.

Calcitonin gene related peptide (hereinafter designates as CGRP) such as human CGRP (h-CGRP), chicken CGRP (c-CGRP) and derivatives thereof are known compounds obtainable by gene manipuration technology or peptide synthesis technology. (Japanese Patent PCT Unexamined Publication No. 60-501562, ibid. 61-500119, PCT WO 85/02840, Japan. Pat. Unexam. Publ. No. 62-29297, U.S. Patent No. 4,697,002, Japan. Pat. Unexam. Publ. No. 63-126894, ibid. 63-258490, ibid. 64-26598, Japan. Pat. PCT Unexam. Publ. No. 3-504503 and Japan. Pat. Unexam. Publ. No. 2-138196)

The h-CGRP is known as a peptide acting on bone metabolism and central nervous system. [Nature, 308(19), 746-748 (1984), FEBS Letters, 183(2), 403 (1985), Neuropeptides, 4, 425-434 (1984) and Nature, 313(3), 54-56 (1984)]. CGRP such as h-CGRP is reported to being absorbed through skin and showing tichogenous activity by action of vasodilation and blood flow stimulation. (Japan. Pat. Unex. Publ. No. 3-44313)

Increasing in survival ratio of skin flap by the vasodilating action and blood stream stimulating action is reported. [Eur. J. Pharmacol., 142, 355-358 (1987), Scand. J. Plastic and Reconstructive Surgery, 23, 11-16 (1989), Brit, J. Plastic Surgery, 43, 447-451 (1990)].

Further h-CGRP is known to have a growth stimulating action of cultured endothelial cells [Regulatory Peptides, 25, 1-9(1989), Proc. Natl. Acad. Sci. U.S.A., 87, 3299-3303 (1990) and J. Natl. Cancer Institute, 27(6), 781-785 (1991)] and angiogenesis [Int. J. Radiation Biol., 60(1-2), 71-72 (1991)].

Furthermore h-CGRP is known to have an anti-inflammatory action [Biochem. Biophys. Res. Comm., 180(3), 1429-1435 (1991)] and inflammation stimulating action [Brit. J. Pharmacol., 103, 1515-1519 (1991) and The J. Immunol., 146, 3424-3430 (1991)].

Desalanyl-deamino-c-CGRP (DADA-c-CGRP) has only known to have strong serum calcium and phosphorous reducing activities (Japan. Pat. Unexam. Publ. No. 63-258490) and a utility for treatment of arteriosclerosis (Japan. Pat. Unexam. Publ. No. 2-229119).

### Problems to be solved by the invention

An experimental model for confirming survival ratio of skin flap is to determine suppression of ischemic necrosis of skin by constriction tion of blood vessel connecting to skin flap and is an experimental model for determining an effect of drug having suppression of vasoconstriction activity or vasodilation activity [Scand. J. Plastic and Reconstructive Surgery, 10, 169-172 (1976)]. The aforementioned report using this experimental model has only indicated to show improved effect of blood flow by h-CGRP and is not to show an effect of wound healing. Blood flow improving effect is merely to prevent deterioration of wound. [Cure and countermeasure for pressure sore, Iyaku Journal Inc., Tokyo, 63 (1993)].

An explanation by pharmacologists indicated that a repair of wound requires formation of granulation tissue filling up the wound stoma and reepithelialization covering the stoma, and is essentially required angiogenesis. Subject having higher activity of angiogenesis shows earlier recovery. Therefore, drugs stimulating angiogenesis promotes wound repairing and is expected to show wound healing effect for decreasing a suffering of disease. Not all of the drugs having stimulating angiogenesis have an action of stimulating wound healing. For example, basic fibroblast growth factor stimulates angiogenesis and formation of granulation tissue in wound (open wound) model using mice, however no stimulation of reepithelialization is observed [J. Exp. Med., 172, 245-251 (1990)], accordingly no satisfactory result for wound healing with earlier filling up the wound stoma is obtained.

The other substances having angiogenic activity, such as angiogenesis factor derived from tumor cells, hydroxy eicosatetraenoic acid and leukotriene D₄, have known. These substances are biological trace substances and are not only difficult to mass-production but also showing side effect such as inflammation and respiratory tract contraction, accordingly are not satisfactory for wound healing remedy.

Inflammation participates early phase of repairing wound and deterioration of inflammation will cause delaying the repair of wound. A medicament showing anti-inflammatory action suppresses angiogenesis and formation of granulation tissue [Brit. J. Pharmacol., 29, 378 (1967)] with delaying repair of wound. Accordingly, considering a complex physiological mechanisms, even if a medicament shows angiogenic activity and anti-inflammatory action essentially the fact that actually the medicament is useful for wound healing remedy can not be determined unless the said medicament effective for the below-mentioned experimental model of wound.

### Means for solving the problems

In the course of studies for developing excellent wound healing remedy, we have found that CGRP of the formula [1] hereinbelow has action of stimulating reepithelialization, formation of granulation tissue and angiogenesis, with showing marked suppression of inflammatory cell infiltration, and is useful for wound healing remedy.

The present invention has completed by the above findings.

An object of the present invention is to provide a wound healing remedy containing CGRP of the formula [1] 
R－CH —— CH₂ —— Y —— Y —— CH₂
|　　　　　　　　　　　　　　　|
CO－ A －Thr－Ala－Thr－NHCH－CO－Val－
Thr－His－Arg－Leu－Ala－ B － C －Leu－
Ser－Arg－Ser－Gly－Gly－ D － E －Lys－
F －Asn－Phe－Val－Pro－Thr－ G －Val－
Gly－Ser－ I －Ala－Phe－NH₂　　　〔1〕
wherein R is Ala-NH-, Ser-NH-, NH₂ or H, Y is S or CH₂, A is Asp or Asn, B is Gly, Asp or Glu, C is Leu or Phe, D is Val or Met, E is Val or Gly, F is Asn, Ser or Asp, G is Asn or Asp, and I is Lys or Glu, or nontoxic salt thereof as an active ingredient.

In the present invention, examples of compound of the formula [1] are, for example, the following CGRP and derivatives thereof in Table 1. Table 1.

**Table 1**

| Sub. | R | Y | A | B | C | D | E | F | G | I |
|---|---|---|---|---|---|---|---|---|---|---|
| 1 | H | S | Asp | Gly | Leu | Val | Val | Asn | Asn | Lys |
| 2 | H | S | Asn | Gly | Leu | Met | Val | Ser | Asn | Lys |
| 3 | Ala-NH- | S | Asn | Gly | Phe | Val | Gly | Asn | Asn | Lys |
| 4 | H | S | Asn | Gly | Phe | Val | Gly | Asn | Asn | Lys |
| 5 | Ala-NH- | S | Asn | Asp | Leu | Val | Gly | Asn | Asn | Lys |
| 6 | H | S | Asn | Asp | Leu | Val | Gly | Asn | Asn | Lys |
| 7 | Ala-NH- | S | Asn | Asp | Phe | Val | Val | Asn | Asn | Lys |
| 8 | H | S | Asn | Asp | Phe | Val | Val | Asn | Asn | Lys |
| 9 | Ala-NH- | S | Asp | Asp | Leu | Val | Val | Asn | Asn | Lys |
| 10 | H | S | Asp | Asp | Leu | Val | Val | Asn | Asn | Lys |
| 11 | Ala-NH- | S | Asn | Glu | Leu | Val | Gly | Asn | Asn | Lys |
| 12 | H | S | Asn | Glu | Leu | Val | Gly | Asn | Asn | Lys |
| 13 | Ala-NH- | S | Asn | Glu | Phe | Val | Val | Asn | Asn | Lys |
| 14 | H | S | Asn | Glu | Phe | Val | Val | Asn | Asn | Lys |
| 15 | Ala-NH- | S | Asp | Glu | Leu | Val | Val | Asn | Asn | Lys |
| 16 | H | S | Asp | Glu | Leu | Val | Val | Asn | Asn | Lys |
| 17 | Ala-NH- | S | Asp | Gly | Leu | Val | Val | Asn | Asn | Lys |
| 18 | Ala-NH- | S | Asn | Asp | Phe | Val | Gly | Asn | Asn | Lys |
| 19 | H | CH₂ | Asn | Asp | Phe | Val | Gly | Asn | Asn | Lys |
| 20 | NH₂ | CH₂ | Asn | Asp | Phe | Val | Gly | Asn | Asn | Lys |
| 21 | NH₂ | CH₂ | Asp | Gly | Leu | Val | Val | Asn | Asn | Lys |
| 22 | Ser-NH- | S | Asn | Gly | Leu | Val | Val | Asp | Asn | Glu |
| 23 | Ser-NH- | S | Asn | Gly | Leu | Met | Val | Ser | Asp | Glu |
| 24 | H | S | Asn | Asp | Phe | Val | Gly | Asn | Asn | Lys |

A salt of CGRP of the formula [1] hereinbefore is a pharmacologically acceptable nontoxic salt. Examples of such the salts are the salt of inorganic acid, for example, hydrochloride and sulfate, and the salt of organic acid, for example, acetate, tartrate, succinate and malate.

Examples of CGRP of the formula [1] are h-CGRP, c-CGRP and derivatives thereof, and can be synthesized by the method disclosed in the specification of Japanese Patent PCT Unexamined Publication No. 61-500119, PCT WO 85/02840, Japan. Pat. Unexam. Publ. No. 62-129297, Japan. Pat. Unexam. Publ. No. 63-126894, ibid. 63-258490, ibid. 64-26598, ibid. No. 2-138196.

CGRP or salt of CGRP thereof of the formula [1] of the present invention can be administered as a wound healing remedy independently or mixed with a pharmacologically acceptable carrier. The composition can be determined depending on its administration route or dosing schedule.

The active ingredient of the present invention is preferably administered parenterally, for example administered intravenously or intramuscularly, or can be administered percutaneously by external preparation. Dosing amount depends upon a type of active ingredient, and is preferably 0.1 µg-200mg parenterally for adult in a day, and most preferably 0.1 µg-20mg in a day.

A type of formulation of the remedy of the present invention is preferably injectable form, but is not limited thereto. Formulation of the remedy is prepared for suitable form of formulation. Injectable form is, for example, prepared by dissolving the active ingredient of the present invention with buffer, isotonic solution, pH adjuster and stabilizing agents in distilled water for injection, filtering through disinfection filter to prepare sterile solution and packing in ampules, or by dissolving the active ingredient with fillers and stabilizing agents in distilled water for injection, filtering through disinfection filter to prepare sterile solution, packing in vials and freeze-drying.

Basic ingredients for preparing external preparations are, for example animal or vegetable oil, mineral oil, ester oil, wax oil, higher alcohols, fatty acids, silicon oil, surface active agents, phospholipids, alcohols, water soluble polymers, clays such as silicate, purified water, and mixture thereof. In the external preparation, keratolytic agents, wetting agents, bactericides such as salicylic acid, resorcin and hexachlorophen, cellular activator, vitamins such as nicotinic acid, vitamin E, vitamin A, pantothenic acid and biotin, hormones, ethynylestradiol, hinokitiol, glycyrrhetines such as glycyrrhetinic acid, fatty acids and amino acids are mixed, if necessary. Further antiseptics, stabilizer, antioxidant, coloring agents and flavor can be added therein.

External preparation of the present invention can be effectuated by penetrating the active ingredient into wound, so that a known compounds having percutaneous absorbability can be combined. Dosage forms of external preparations including active ingredients of the present invention can not be limited, and is, for example tonic, lotion, cream, aerosols, ointments or cataplasmas.

### Examples

Following examples illustrate the present invention but are not construed as limiting. Indication of the active ingredient of the present invention in the examples is based upon the numbers of the compound in Table 1 as indicated by [compound (No.)].

### Example 1

### Test for therapeutic effect on wound healing (1)

### (1) Method:

Therapeutic effect for wound healing was tested according to a method of Yamashita et al. [Pharmacometrics (Oyoyakuri), 37, 313-327 (1989)] in rats. A grained right femur of pentbarbital anesthesized rat set on fixater were pressed (1.5 kg/cm²) by lower end of disposable syringe (diameter 16.5mm), for 8 hours in a day for 5 days. Acrylic plate, 1cm square, was placed between the femur and syringe.

Evaluation of the active ingredient was made by macroscopically and histopathologically. Macroscopic observation was made by calculating a number of days required for healing with complete reepithelialization of pressure sore section. Histopathological evaluation was made by collecting a skin of pressure sore section at the healing day, fixing the skin in 10% buffered formal in solution, embedding in paraffin, sectioning, staining with H.E., observing microscopically and classifying three grades (±, +, ++) of the degree of reepithelialization, granulation tissue and formation angiogenesis.

Test medicament was administered continuously 1 × 10 ⁻⁸mole/kg in a day by using miniosmotic pump (Alzet Inc.). A miniosmotic pump was embeded subcutaneously near the pressure sore. The active ingredient administered group (experimental group) was administered of DADA-c-CGRP [compound (24)] dissolved in 0.1% BSA physiological salt solution. Control group was administered 0.1% BSA physiological salt solution.

### (2) Results

In the macroscopical observation healing date of 24 days was observed in the control group, and that of 21 days was observed in the experimental group, accordingly 3 days shortenings were observed. In the histpathological findings, as shown in Table 2, experimental group showed excellent extension of thick stratum spinosum epiderdimis, stimulation of reepithelialization and good formation of granulation tissue with many infiltration of fibroblasts. Increase in capillary number was excellently observed.

**Table 2**

| Test medicament | No. of animals | reepithelialization | | | granulation tissue formation | | | angiogenesis | | |
|---|---|---|---|---|---|---|---|---|---|---|
| | | ± | + | ++ | ± | + | ++ | ± | + | ++ |
| Control | 6 | 3 | 2 | 1 | 3 | 3 | 0 | 4 | 1 | 1 |
| Test group | 5 | 0 | 2 | 3 | 0 | 2 | 3 | 0 | 1 | 4 |

Results hereinabove indicate strong effect for wound healing of the active ingredient [compound (24)] of the present invention.

### Example 2

### Test for therapeutic effect on wound healing(2)

### (1) Method:

Therapeutic effect for wound healing was tested according to a method of Yamashita et al. [Pharmacometrics (Oyoyakuri), 37, 313-327 (1989)] in rats. A grained right femur of pentbarbital anesthesized rat set on fixater were pressed (1.5 kg/cm²) by lower end of disposable syringe (diameter 16.5mm), for 8 hours in a day for 5 days. Acrylic plate, 1cm square, was placed between the femur and syringe.

Evaluation of the active ingredient was made by macroscopically and histopathologically. Macroscopic observation was made by calculating a number of days required for healing with complete reepithelialization of pressure sore section. Histopathological evaluation was made by collecting a skin of pressure sore section at the healing day, fixing the skin in 10% buffered formal in solution, embedding in paraffin, sectioning, staining with H.E., observing microscopically and classifying three grades (±, +, ++) of the degree of reepithelialization, granulation tissue formation, angiogenesis and infiltration of inflammatory cells.

Test medicament of the ointment prepared by containing 50 µg of the compound (24) in ointment base (plastibase) 1g was administered to the pressure sore section with 100mg in a day (experimental group). Control group was administered ointment base only. Necrotic part of pressure sore section was removed by surgically.

### (2) Results

In the macroscopical observation, healing date of 22 days was observed in the control group, and that of 16 days was observed in the experimental group, accordingly 6 days shortenings of healing were observed. In the histpathological findings, as shown in Table 3, the experimental group showed excellent extension of thick stratum spinosum epiderdimis, stimulation of reepithelialization and good formation of granulation tissue with many infiltration of fibroblasts. Increase in angiogenesis and suppression of infiltration of inflammatory cells were excellently observed.

**Table 3**

| Test medicament | No. of animals | reepithelialization | | | granulation tissue formation | | | angiogenesis | | | inflammatory cell infiltration | | |
|---|---|---|---|---|---|---|---|---|---|---|---|---|---|
| | | ± | + | ++ | ± | + | ++ | ± | + | ++ | ± | + | ++ |
| Control | 6 | 2 | 3 | 1 | 3 | 3 | 0 | 4 | 1 | 1 | 1 | 1 | 4 |
| Test group | 6 | 0 | 2 | 4 | 0 | 2 | 4 | 0 | 1 | 5 | 5 | 1 | 0 |

Results hereinabove indicate strong effect for wound healing of the active ingredient [compound (24)] of the present invention.

As clearly observed in the results (1) and (2) hereinabove, the compound (24) of the active ingredient was shown to have strong activity for wound healing by the present invention, accordingly clinical usefulness of the active ingredient is strongly expected.

The active ingredient DADA-c-CGRP [compound (24)] has no amino group in its N-terminal and is hardly decomposed by aminopeptidase, so that it is quite stable in vivo as compared with other CGRP having N-terminal amino group, and shows strong effect for wound healilng.

### Example 3.

### Toxicological studies:

### (1) Method

The compound(24) dissolved in 0.1% BSA physiological salt solution was continuously administered 1 × 10 ⁻⁷mole/kg (total dose 10.5mg/kg/4 weeks) by using miniosmotic pump (Alzet Inc.). A miniosmotic pump was embeded subcutaneously in the femur of rat, and result of mortality was determined after 4 weeks,

### (2) Results

No death was observed by administration of the compound (24). No abnormal symptoms were observed.

The compound(24) was confirmed to be quite safety.

### Example 4

### Preparation of injections

The compound (24) 100mg, mannitol 10g and stabilizing agent were dissolved in distilled water for injection, passed through disinfection filter, filled each 2ml thereof in a vial, and lyophilized to obtain the injection of the compound (24) 100µg/vial. The preparation is administered with dissolving in physiological salt solution before use.

### Example 5

### Preparation of injections

The compound (24) 60mg, buffer, isotonic solution, pH adjuster and stabilizing agent were dissolved in 6 lit. distilled water for injection., passed through disinfection filter, filled each 1ml thereof in an ampule, and sealed by fusion to obtain the injection of the compound (24) 10µg/ampule.

### Example 6

### Preparation of ointment

| | |
|---|---|
| The compound(24) | 80.0mg |
| scuarane | 10.0g |
| white petrolatum | 8.0g |
| cetostearyl alcohol | 8.0g |
| glyceryl monostearate | 2.0g |
| polyoxyethylene monostearate | 1.0g |
| methyl parahydroxybenzoate | 0.2g |
| propyl parahydroxybenzoate | 0.1g |
| 1,3-butylene glycol | 2.5g |
| purified water | in proper |
| Total | 100.0g |

Oil phase consisting of scuarane, white petrolatum, cetostearyl alcohol, glyceryl monostearate and polyoxyethylene monostearate were melted at 75°C. Water phase consisting of methyl parahydroxybenzoate, propyl parahydroxybenzoate, 1,3-butylene glycol, the compound (24) and purified water were dissolved at 60°C. The oil phase of being cooled to 60°C was added to the water phase, homogenized by agitating with the homomixer, and cooled to room temperature with stirring to obtain the ointment containing the components hereinabove,

### Example 7

### Test for therapeutic effect on wound (3)

### (1) Method

Therapeutic effect for wound was tested according to a method of Yamashita et al. [Pharmacometrics (Oyoyakuri), 37, 313-327 (1989)] in rats. A grained right femur of pentbarbital anesthesized rat set on fixater were pressed (1.5kg/cm²) by lower end of disposable syringe (diameter 16.5mm), for 8 hours in a day for 5 days. Acrylic plate, 1cm square, was placed between the femur and syringe.

Evaluation of the active ingredient was made by macroscopically and histopathologically. Macroscopic observation was made by calculating a number of days required for healing with complete of reepithelialization of pressure sore section. Histopathological evaluation was made by collecting a skin of pressure sore section at the healing day, fixing the skin in 10% buffered formal in solution, embedding in paraffin sectioning, staining with H.E., observing microscopically and classifying three grades (±, +, ++) of the degree of reepithelialization, granulation and vascularization.

Test medicament was administered continuously 1 × 10 ⁻⁸mole/kg in a day by using miniosmotic pump (Alzet Inc.). A miniosmotic pump was embeded in the subcutaneously near the pressure sore. The active ingredient administered group (experimental group) was administered the test medicament of a solution of the compound (17) dissolved in 0.1% BSA physiological salt solution. Control group was administered 0.1% BSA physiological salt solution.

### (2) Results

In the macroscopical observation healing date of 23 days was observed in the control group, and that of 20 days was observed in the experimental group, accordingly 3 days shortenings were observed. In the histpathological findings, as shown in Table 4, the experimental group showed excellent extension of thick stratum spinosum epiderdismis, stimulation of reepithelialization and good formation of granulation tissue with many infiltration of fibroblasts. Increase in angiogenesis was excellently observed.

**Table 4**

| Test medicament | No. of animals | reepithelialization | | | granulation tissue formation | | | angiogenesis | | |
|---|---|---|---|---|---|---|---|---|---|---|
| | | ± | + | ++ | ± | + | ++ | ± | + | ++ |
| Control | 6 | 3 | 2 | 1 | 3 | 3 | 0 | 4 | 1 | 1 |
| Test group | 6 | 0 | 3 | 3 | 0 | 2 | 4 | 0 | 1 | 5 |

Result hereinabove indicate strong effect for wound healing of the active ingredient [compound (17)] of the present invention.

### Example 8

### Test for therapeutic effect on wound healing (4)

### (1) Method:

Therapeutic effect for wound healing was tested according to a method of Yamashita et al. [Pharmacometric (Oyoyakuri), 37, 313-327 (1989)] in rats. A grained right femur of pentbarbital anesthesized rat set on fixater were pressed (1.5kg/cm²) by lower end of disposable syringe (diameter 16.5mm), for 8 hours in a day for 5 days. Acrylic plate, 1cm square, was placed between the femur and syringe.

Evaluation of the active ingredient was made by macroscopically and histopathologically. Macroscopic observation was made by calculating a number of days required for healing with complete reepithelialization of pressure sore section. Histopathological evaluation was made by collecting a skin of pressure sore section at the healing day, fixing the skin in 10% buffered formal in solution, embedding paraffin, sectioning, staining with H.E., observing microscopically and classifying three grades (±, +, ++) of the degree of reepithelialization, granulation tissue formation, angiogenesis and infiltration of inflammatory cells.

Test medicament of the ointment prepared by containing 50 µg of the compound (17) in ointment base (plastibase) 1g was administered to the pressure sore section with 100mg in a day (experimental group). Control group was administered oinyment base only. Necrotic part of the pressure sore was removed by surgically.

### (2) Results

In the macroscopical observation healing date of 22 days was observed in the control group, and that of 17 days was observed in the experimental, accordingly 5 days shortenings of healing were observed. In the histpathological findings, as shown in Table 5, the experimental group showed excellent extension of thick stratum spinosum epiderdimis, stimulation of reepithelialization and good formation of granulation tissue with many infiltration of fibroblasts. Increase in angiogenesis and suppression of infiltration of inflammatory cells were excellently observed.

**Table 5**

| Test medicament | No. of animals | reepithelialization | | | granulation tissue formation | | | angiogenesis | | | inflammatory cell infiltration | | |
|---|---|---|---|---|---|---|---|---|---|---|---|---|---|
| | | ± | + | ++ | ± | + | ++ | ± | + | ++ | ± | + | ++ |
| Control | 6 | 1 | 4 | 1 | 3 | 2 | 1 | 4 | 1 | 1 | 1 | 2 | 3 |
| Test group | 6 | 0 | 2 | 4 | 0 | 3 | 3 | 0 | 1 | 5 | 4 | 1 | 1 |

Results hereinabove indicate strong effect for wound healing of the active ingredient [compound (17)] of the present invention.

As clearly observed in the test results (1) and (2) hereinabove, the compound (17) of the active ingredient was shown to have strong activity for wound healing by the present invention, accordingly clinical usefulness of the active ingredient is strongly expected.

### Example 9.

### Toxicological studies:

### (1) Method

The compound (17) dissolved in 0.1% BSA physiological salt solution was continuously administered 1 × 10 ⁻⁷mole/kg (total dose 10.5mg/kg/4 weeks) by using miniosmotic pump (Alzet Inc.). A miniosmotic pump was embeded subcutaneously in the femur of rat, and result of mortaliyu was determined after 4 weeks.

### (2) Results

No death was observed by administration of the compound (17). No abnormal symptoms were observed.

The compound (17) was confirmed to be quite safety.

### Example 10

### Preparation of injections

The compound (17) 100mg, mannitol 10g and stabilizing agent were dissolved in distilled water for injection, passed through disinfection filter, filled each 2ml thereof in a vial, and lyophilized to obtain the injection of the compound (17) 100µg/vial. The preparation is administered with dissolving in physiological salt solution before use.

### Example 11

### Preparation of injections

The compound (17) 60mg, buffer, isotonic solution, pH adjuster and stabilizing agent were dissolved in 6 lit. distilled water for injection., passed through disinfection filter, filled each 1ml thereof in an ampule, and sealed by fusion to obtain the injection of the compound (17) 10µg/ampule.

### Example 12

### Preparation of ointment

| | |
|---|---|
| Compound (17) | 80.0mg |
| scuarane | 10.0g |
| white petrolatum | 8.0g |
| cetostearyl alcohol | 8.0g |
| glyceryl monostearate | 2.0g |
| polyoxyethylene monostearate | 1.0g |
| methyl parahydroxybenzoate | 0.2g |
| propyl parahydroxybenzoate | 0.1g |
| 1,3-butylene glycol | 2.5g |
| purified water | in proper |
| Total | 100.0g |

Oil phase consisting of scuarane, white petrolatum, cetostearyl alcohol, glyceryl monostearate and polyoxyethylene monostearate were melted at 75°C. Water phase consisting of methyl parahydroxybenzoate, propyl parahydroxybenzoate, 1,3-butylene glycol, the compound (17) and purified water were dissolved at 60°C. The oil phase of being cooled to 60°C was added to the water phase, homogenized by agitating with the homomixer, and cooled to room temperature with stirring to obtain the ointment containing the components hereinabove.

### Effect of the invention

As clearly shown in the above, the active ingredient of the present invention is, when administered intravenously, intramuscularly, subcutaneously or externally to the experimental animal pressure sore model, the most intractable in the wound, effective for shortening the healing days by stimulating reepithelialization of wound part, formation of granulation tissue of wound part, and increase in angiogenesis with markedly suppressing infiltration of inflammatory cells, therefore is excellently useful for wound remedy.

## Claims

1. A wound healing remedy containing calcitonin gene related peptide of the formula 
R-CH —— CH₂ ——— Y ——— Y —— CH₂
  |                                                    |
  CO- A -Thr-Ala-Thr-NHCH-CO-Val-
Thr-His-Arg-Leu-Ala- B - C -Leu-
Ser-Arg-Ser-Gly-Gly- D - E -Lys-
  F -Asn-Phe-Val-Pro-Thr- G -Val-
Gly-Ser- I -Ala-Phe-NH₂
 wherein R is Ala-NH-, Ser-NH-, NH₂ or H, Y is S or CH₂, A is Asp or Asn, B is Gly, Asp or Glu, C is Leu or Phe, D is Val or Met, E is Val or Gly, F is Asn, Ser or Asp, G is Asn or Asp, and I is Lys or Glu, or nontoxic salt thereof as an active ingredient.

2. A wound healing remedy containing calcitonin gene related peptide of the formula 
R-CH —— CH₂ ——— Y ——— Y —— CH₂
  |                                                    |
  CO- A -Thr-Ala-Thr-NHCH-CO-Val-
Thr-His-Arg-Leu-Ala- B - C -Leu-
Ser-Arg-Ser-Gly-Gly- D - E -Lys-
  F -Asn-Phe-Val-Pro-Thr- G -Val-
Gly-Ser- I -Ala-Phe-NH₂
 wherein R is Ala-NH-, Ser-NH-, NH₂ or H, Y is S or CH₂, A is Asp or Asn, B is Gly, Asp or Glu, C is Leu or Phe, D is Val or Met, E is Val or Gly, F is Asn, Ser or Asp, G is Asn or Asp, and I is Lys or Glu, in which when Y is S, A is Asp, B is Gly, C is Leu, D is Val, E is Val, F is Asn, G is Asn, and I is Lys, R is not Ala-NH-, or nontoxic salt thereof as an active ingredient.

3. A wound healing remedy containing calcitonin gene related peptide of the formula 
R－CH ── CH₂ ─── Y ─── Y ── CH₂
  |                                    |
  CO－ A －Thr－Ala－Thr－NHCH－CO－Val－
Thr－His－Arg－Leu－Ala－ B － C －Leu－
Ser－Arg－Ser－Gly－Gly－ D － E －Lys－
 F －Asn－Phe－Val－Pro－Thr－ G －Val－
Gly－Ser－ I －Ala－Phe－NH₂
 wherein R is Ala-NH-, Ser-NH-, NH₂ or H, Y is S or CH₂, A is Asp or Asn, B is Gly, Asp or Glu, C is Leu or Phe, D is Val or Met, E is Val or Gly, F is Asn, Ser or Asp, G is Asn or Asp, and I is Lys or Glu, in which when Y is S, A is Asn, B is Asp, C is Phe, D is Val, E is Gly, F is Asn, G is Asn, and I is Lys, R is not H, or nontoxic salt thereof as an active ingredient.

4. A wound healing remedy containing calcitonin gene related peptide of the formula 
H-Ala-Cys-Asp-Thr-Ala-Thr-Cys-Val-
Thr-His-Arg-Leu-Ala-Gly-Leu-Leu-
Ser-Arg-Ser-Gly-Gly-Val-Val-Lys-
Asn-Asn-Phe-Val-Pro-Thr-Asn-Val-
Gly-Ser-Lys-Ala-Phe-NH₂
 or nontoxic salt thereof as an active ingredient.

5. A wound healing remedy containing calcitonin gene related peptide of the formula 
CH₂ — CH₂ ——— S ——— S —— CH₂
|                                                    |
CO−Asn−Thr−Ala−Thr−NHCH−CO−Val−
Thr−His−Arg−Leu−Ala−Asp−Phe−Leu−
Ser−Arg−Ser−Gly−Gly−Val−Gly−Lys−
Asn−Asn−Phe−Val−Pro−Thr−Asn−Val−
Gly−Ser−Lys−Ala−Phe−NH₂
 or nontoxic salt thereof as an active ingredient.
